# EUROPEAN PATENT SPECIFICATION

(11) **EP 3 052 645 B1**
(45) Date of publication and mention of the grant of the patent: **09.01.2019**
(21) Application number: 14781126.9
(22) Date of filing: 29.09.2014
(51) Int. Cl.: C12Q 1/68, C12N 15/10

(54) **DNA-ADAPTER-MOLECULES FOR THE PREPARATION OF DNA-LIBRARIES AND METHOD FOR PRODUCING THEM AND USE**
DNA-ADAPTERMOLEKÜLE ZUR HERSTELLUNG VON DNA-BIBLIOTHEKEN UND HERSTELLUNGS- UND VERWENDUNGSVERFAHREN DAFÜR
MOLÉCULES D'ADAPTATEUR ADN POUR LA PRÉPARATION DE BIBLIOTHÈQUES D'ADN ET LEURS PROCÉDÉS DE PRODUCTION ET D'UTILISATION

(30) Priority: 30.09.2013 EP 13186776
(43) Date of publication of application: 10.08.2016
(73) Proprietor: Qiagen GmbH, 40724 Hilden (DE)
(72) Inventor: HAHN, Peter, 51429 Bergisch Gladbach (DE); AZZAWI, Alexander, 42699 Solingen (DE); GRÜNEFELD, Peter, 40723 Hilden (DE)
(74) Representative: Kailuweit & Uhlemann Patentanwälte Partnerschaft mbB
(86) International application number: PCT/EP2014/070769
(87) International publication number: WO 2015/044412

(56) References cited:
- EP-A1- 1 574 585
- WO-A1-97/46704
- WO-A2-2011/133876
- US-B1- 6 528 257

## Description

The invention relates to DNA-adapter-molecules for the preparation of DNA-libraries and methods for producing them and their use. The invention is useful for the application in molecular biology, in particular for Next Generation Sequencing and/or Library Multiplexing.

### State of the art:

Next Generation Sequencing (NGS) is a modern technology for determining the sequence of nucleobases of a DNA- or RNA-sequence. The advantages of NGS (speed, economical effectiveness, accurateness) are leading to a growing distribution at all academic institutions that deal with the analysis of genetic information and its implementation. Several global companies offer solutions for NGS, including Roche, Life Technologies and Illumina.

For sequencing a number of samples in one run, barcodes of known sequence can be added to defined samples and be used for assigning sequences to samples after the sequencing.

Methods, compositions and kits for multiplex sequencing (simultaneously sequencing a number of different samples) are disclosed in WO 2011156529 A2. A plurality of target polynucleotides from two or more different samples is sequenced in one reaction chamber and the sample from which each of the sequenced target polynucleotides is derived from is identified via barcodes.

Another document discloses sequencing methods with improved sample throughput is WO 2008061193 A2. Herein adapter sequences (referred to as tag sequences) are linked to samples which are then being mixed and sequenced.

WO 2013033721 A1 discloses methods for optimizing barcode design for multiplex DNA sequencing.

US 2013059762 A1 provides methods, compositions, kits, systems and apparatus that are useful for multiplex PCR of one or more nucleic acids present in a sample. In particular, various target-specific primers are provided that allow for the selective amplification of one or more target sequences. Therefore adapters are ligated to target sequences in a blunt-ended ligation reaction.

During the preparation of DNA libraries for Next Generation Sequencing (NGS) high molecular dsDNA in the majority of cases is fragmented via physical or chemical methods and adapter sequences specific to the sequencing platform are being ligated to the generated DNA molecules. The adapter sequences in general contain binding sites for primers and might contain barcode sequences, that allow to sequence multiple barcoded DNA libraries in a mixture (multiplexing) and to subsequently assign the sequencing information about the respective barcode to the original sample.

Before this ligation of the adapter sequences to the fragments can be carried out, the DNA endings which are formed randomly and unforeseeable during the fragmentation have to be repaired. Overhanging 3' ends are excised and overhanging 5' ends are filled up to a double strand by a polymerase. Subsequently 5' ends of the fragments are being phosphorylated by a kinase. Afterwards, unphosphorylated DNA-adapters, which are blunt ended at one side to allow the ligation there and which have a 3' overhang at the other side to avoid a ligation there, are ligated to the generated DNA molecules with the aid of a ligase. Since the ligation of unphosphorylated adapter sequences is not leading to the formation of phosphodiester linkages at the 5' end of the adapter, thus leaving a nick in the DNA double strand after ligation, the missing phophodiester linkage subsequently needs to be closed by an enzyme, which is able to conduct nick translation.

Commonly used adapter sequences cannot be added to the end repair mix, since the enzymes in the end repair mix would modify them in a way that the directionality of the ligation gets lost and adapter dimers and oligomers would be formed.

### Objective

It is the objective of the present invention to modify adapter sequences in a way that they can already be added to the fragmented DNA at the beginning of the library preparation. Another objective of the invention is to avoid the formation of adapter dimers and oligomers.

### Invention

The present invention discloses DNA-adapter-molecules, comprising a double-stranded polynucleotide molecule, whereat
**a.** the 5' end of the first strand is modified in a way, that the first nucleotide does not contain a free hydroxyl group and not a free phosphate at the 5' position, so that no binding site for kinases is available,
**b.** the 3' end of the first strand is modified in a way that the last nucleotide does not contain a free hydroxyl group at the 3' position, so that no ligation can occur,
**c.** the 5' end of the reverse strand is modified in a way, that the first nucleotide does not contain a free hydroxyl group and not a free phosphate at the 5' position, so that no binding site for a kinase is available, and
**d.** the 3' end of the reverse strand features a free hydroxyl group (at the 3' position of the last nucleotide).

First strand and reverse strand are annealed to each other by complementary base pairing, without any overhang (blunt ends). Preferably the nucleotides within the double-strand, other than the above mentioned terminal nucleotides at the 3' and 5' ends, are non-modified. However it is not excluded that the double strand contains nucleotides with enhanced stability against nucleases, in particular nucleotides with a modified backbone, like phosphorothioates. Preferably both strands have exactly the same length (with an identical number of nucleotides), wherein the nucleotide sequence of the reverse strand is the reverse complement of the nucleotide sequence of the first strand.

Advantageously, the DNA-adapter-molecules according to the invention can be added during DNA library preparation directly to the fragmented DNA. Opposite to the state of the art here is no need to inactivate the end-repair enzymes before adding DNA-adapter-molecules according to the invention and before adding the ligase. Since the adapter-molecules according to the invention are blunt ended, they are not a substrate for polymerases with 5'->3' polymerase activity and 3'->5' exonuclease activity, like T4 DNA Polymerase, T7 DNA Polymerase or Pfu polymerase.

Further, the DNA-adapter-molecules according to the invention are advantageously designed in a way that enzymatic ligation can only occur in one direction (at the free hydroxyl group of the 3' end of the reverse strand).

The term "free hydroxyl group" as used herein refers to -OH or the deprotonated -O⁻. The term "free phosphate" is used herein to describe a deprotonated phosphate (-OPO₃²⁻), mono- or dihydrogenphosphate.

The term "polymerase" as used herein refers to a DNA dependent DNA polymerase, preferably a polymerase with 5'->3' polymerase activity and 3'->5' exonuclease activity and preferably no 5'->3' exonuclease activity. Preferred Polymerases are enzymes, which are able to create blunt ends, like T4 Polymerase , T7 DNA Polymerase or Pfu polymerase.

The term "kinase" as used herein refers to a polynucleotide 5'-hydroxyl-kinase that catalyzes the addition of a phosphate to a free 5'-hydroxyl end of a polynucleotide molecule. Preferred kinases are T4 kinase or T7 kinase.

A "polynucleotide molecule" as used herein is a biopolymer composed of 13 or more nucleotide monomers covalently bonded in a chain.

The DNA-adapter-molecule according to the invention preferably additionally contains binding sites for amplification and sequencing primers and preferably a barcode sequence.

The barcode sequence has preferably a length of three to 20, more preferably four to eight base pairs. Preferably the barcode sequence is located close to the 5' end of the first strand. The DNA-adapter-molecule has preferably a length of 20 to 90, more preferred of 30 to 70, most preferred of 40 to 60 base pairs.

By the expression that the first and/or last nucleotide does not contain a free hydroxyl group it is meant that the DNA-adapter-molecule according to the invention is modified in a way that the normally occurring hydroxyl or phosphate at 5' ends of both strands and the normally occurring hydroxyl at the 3' end of the reverse strand are replaced by other groups, here referred to as "terminating groups", which result in a modified nucleotide that is not a substrate for the respective enzymes mentioned above. Preferably these terminating groups are independently chosen from hydrogen, substituted or unsubstituted alkyl, alkoxy amino and other known chain terminators. It is not excluded that these terminating groups can themselves contain free hydroxyl groups or even a free phosphate, as these will be not substrates for the enzymes.

The DNA-adapter-molecule according to the invention is preferably modified in that the 5' ends of both strands and the 3' end of the first strand contain chain terminators.

Chain terminators for the 3' end as well as the 5' end are well known to a person skilled in the art. At the 5' end of the reverse strand and the 3' end of the first strand any possible chain terminator can be chosen to block the free 3'-OH or 5'-OH respectively.

Preferred examples of chain terminators for the 3' end of the first strand are 2'3'-dideoxynucleosides (formula 1) or 3'-modified deoxynucleotides, preferably as depicted in formula 2: 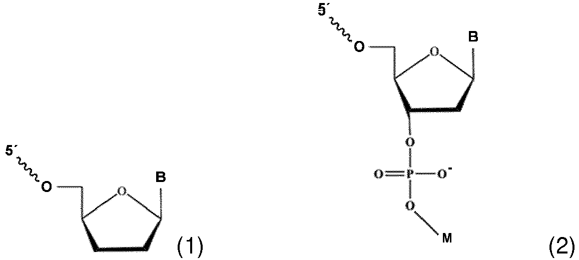 with B = nucleobase, preferably selected from Adenine (A), Guanine (G), Cytosine (C) and Thymine (T) and M selected from NH₂-L with L is preferably selected form linear or branched C1 to C6 alkyl, preferably C3 or C4 alkyl,
wherein 5' 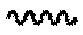 O is the covalent bond to the ongoing nucleotide sequence of the first strand of the DNA-adapter-molecule in 3' → 5' direction.

Preferred examples of chain terminators for the 5' end are etherified deoxynucleotides (formula 3), 4'-amino-deoxynucleotides (formula 4) and 5'-amino-deoxynucleotides (formula 5, like: 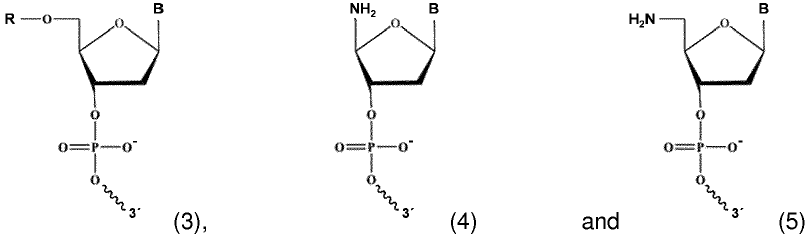 with B = nucleobase selected as above and preferably R = C1 to C3 alkyl, more preferably CH₃,
wherein O 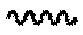 3' is the covalent bond to the ongoing nucleotide sequence of the first strand or reverse strand of the DNA-adapter-molecule in 5'→3' direction.

Preferably the 5' ends of both strands are independently from each other modified by 5'-OMe-deoxynucleotides,most preferred 5'-OMe deoxythymidine (5'-OMeT), terminal 5'-C3-spacer-modifications and/or 5'-D-spacer-modifications. This modifiation results in the fact, that phosphorylation via polynucleotide kinases, especially via T4 polynucleotide kinase, cannot be conducted at the modified 5' end. Advantageously, the 5' ends of both strands remain unphosphorylated to avoid the formation of adapter dimers and oligomers.

Preferably, a 5'-C3-spacer or a 5'-D-spacer is designed as follows 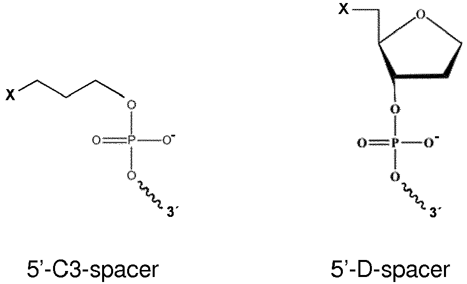 wherein X is selected from -H, -OH, -ORₓ and halogen, preferably X is -OH or -ORₓ, more preferably X is -OH,
wherein Rₓ is an optionally substituted and/or branched C1 to C3 alkyl residue, more preferably Rₓ is CH₃,
wherein O 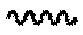 3' is the covalent bond to the ongoing nucleotide sequence of the first strand or reverse strand of the DNA-adapter-molecule in 5'→3' direction. 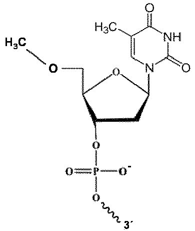 5'-OMe deoxythymidylate (5'-O-methyl deoxythymidine monophosphate, here also referred to as 5'-OMeT).

Preferably the 5' end of the first strand of the DNA adapter molecule is modified by a 5'-spacer or a 5' etherified deoxynucleotide (preferably according to formula 3), preferably a 5'-OMethyl deoxynucleotide. The term spacer means a hydrocarbon residue with preferably one to six carbon atoms, preferably an alkdiyl group with 2 to 4 carbon atoms, most preferred linear C3 (5'-C3-spacer). The modification of the 5' end of the first strand of the DNA adapter molecule results in the fact that phosphorylation, in particular via T4 polynucleotide kinase, is not possible. Furthermore, the preferred modifications have the least influence (sterical hindrance) on the conformation of the DNA double strand to allow an efficient ligation of the reactive free 3'-OH of the reverse strand.

The most preferred 5'-OMe deoxynucleotide is 5'-OMe deoxythymidylate (5'-OMeT). Also possible are modifications by 5'-OMe deoxycytidylate (5'-O-methyl deoxycytidine monophosphate), 5'-OMe deoxyadenylatee (5'-O-methyl deoxyadenosine monophosphate), or 5'-OMe deoxyguanylate (5'-O-methyl deoxyguanosine monophosphate). For the 5'-OMe deoxynucleotide modification (or 5'-amino-deoxynucleotide) an additional complementary deoxynucleotide is added to the 3'-end of the reverse strand of the DNA adapter molecule so that the modified nucleotide at the 5'-end of the first strand can form a normal, non-stabilized base pair with this complementary nucleotide to not interfere the following nick repair. If for example the modification of the 5' end of the first strand is 5'-OMeThymidine (or 5'-aminodeoxythymindine mono phosphate), the complementary nucleotide is a deoxyadenine.

When used for library preparation the DNA-adapter-molecule according to the invention is ligated only with the 3'- end of the reverse strand to a 5'-phosphorylated end of the first strand of a DNA fragment. Between the 5'-end of the first strand of the DNA adapter and the 3'-OH of the reverse strand of the DNA fragment remains a nick (due to the 5' end modification of the first strand of the DNA adapter molecule, the 5' end does not contain a free phosphate). The nick is preferably subsequently repaired by a nucleotide excision repair (here also called nick repair) with a Polymerase with 5'->3' exonuclease activity and 5'-> 3' polymerase activity (like DNA polymerase I) excising and replacing the modified nucleotide at the 5'-end (preferably 5'-OMeT) by a unmodified nucleotide (preferably deoxythymidine monophosphate). The ligase preferably still present and active in the sample then ligates the free 3'-hydroxyl group of the reverse strand of the DNA fragment to the repaired 5'-end of the first strand of the DNA adapter.

The 3' end of the first strand of the DNA-adapter-molecule according to the invention is preferably modified by 3'-C3-spacer or 3'-amino-modifier. That modified 3' end is blocked for ligase and no junction to 5' phosphorylated DNA, preferably dsDNA molecules can occur. 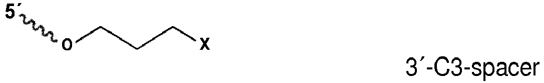 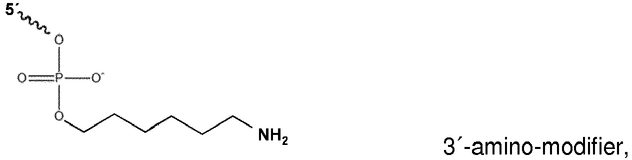 wherein X is selected from -H, -OH, -ORₓ and halogen, preferably X is -OH or -ORₓ, more preferably X is -OH,
wherein Rₓ is an optionally substituted and/or branched C1 to C3 alkyl residue, more preferably Rₓ is CH₃,
wherein 5' 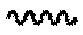 O is the covalent bond to the ongoing nucleotide sequence of the first strand of the DNA-adapter-molecule in 3'→5' direction.

Also part of the invention is a method for the production of DNA-adapter-molecules, comprising a double-stranded DNA molecule with the following steps:
a. modification of the 5' end of the first strand in a way, that no binding site for kinase, especially no free hydroxyl group, and no free phosphate is available,
b. modification of the 3' end of the first strand in a way that no free hydroxyl group is available and that no bond with 5'-phosphorylized dsDNA can be formed,
c. modification of the 5' end of the reverse strand in a way, that no binding site for a kinase, especially no free hydroxyl group, and no free phosphate is available,
whereat the steps a-c can occur in arbitrary order.

The double strands are normally obtained by annealing two single strands synthesized by standard oligonucleotide synthesis. In general the above mentioned modifications are introduced by using already pre-modified building blocks for oligonucleotide synthesis for the respective 3' and 5' ends. However the term modification also includes the (less preferred) possibility to modify the respective 3' and 5' ends after oligonucleotide synthesis, e. g. transformation of a standard 5'-Thymidine (more exactly deoxythymidine monophosphate) with a free 5'-OH to a methyl ether to obtain 5'-OMeT.

Preferred modifications are described above.

Another part of the invention is a method for the generation of a DNA library preferably comprising the following steps:
1. fragmentation of double stranded DNA, preferably by physical or chemical or enzymatic methods or a combination thereof,
2. end repair of the fragmented double stranded DNA, preferably with the aid of a DNA polymerase with 3'->5'exonuclease and 5'->3' polymerase activity, like T4-DNA polymerase,
3. phosphorylation of 5' ends of the fragmented double stranded DNA, preferably by a polynucleotide kinase, like T4 polynucleotide kinase,
4. ligation of DNA-adapter-molecules according to the invention to the fragmented, end repaired and 5' phosphorylated double stranded DNA, whereat the DNA-adapter-molecules according to the invention are added before step 2 and preferably no inactivation of enzymes (in particular end repair enzymes) is performed before ligation.

The enzymes are added in suitable buffers known in the state of the art. Steps 2 and 3 can be performed in parallel, in particular by adding a mix of polymerase and kinase. Both enzymes together are also named herein "end repair enzymes". The mix is also named "end repair enzyme mix" respectively. The polymerase used for end repair and the kinase perform well in the same buffer, preferably comprising magnesiumand a buffering agent, like TRIS-HCI and preferably with a pH between 7 and 8. This buffer is further also referred to as "end repair buffer". For end repair deoxynucleoside triphosphates (preferably dATP, dTTP, dGTP and dCTP) are also added either to the "end repair buffer" or separately.

The DNA adapter-molecules according to the invention are built up as explained above, preferably including one or several preferred features described above. Preferably the DNA adapter-molecules added before step 2 contain bar codes sequences. In step 1 several individual DNA probes can be treated in parallel in individual compartments of a device. Preferably to every individual DNA probe a DNA adapter-molecule with different bar codes is added. For next generation sequencing the samples are generally pooled after ligation of bar codes. These bar codes allow assigning sequences to samples after the sequencing.

In principle the barcode adapters can be added at every time after step 1 and before or during step 4.

Advantageously the DNA-adapter-molecules according to the invention can already be added to the fragmented DNA, before the end repair and the phosphorylation of 5' ends is performed. This has the advantages that only in step 1 are added individualizes probes, which are fragmented DNA and DNA-adapter-molecules according to the invention with barcode. This allows the automation of the subsequent steps, e. g. with a pipetting robot, as in steps 2 to 4 preferably the same substances and volumes are added to all samples.

In protocols known in the state of the art small volumes (2 *µ*l or less) of individualized bar code adaptors have to be added manually to the sample directly before ligation, after end repair and inactivation of end repair enzymes. This error prone pipetting of small volumes can be avoided according to the invention.

However in the invention the barcode adapters can be added to each sample after step 1 and the following steps can be completely carried out from a pipetting robot. The robot can be supplied with universal End-Repair- and Ligation-Mixes and just has to add identical preset volumes to each sample. This minimizes inaccuracy and a loss of Reaction Volume. Furthermore the robot does not need to change the pipetting tip after each pipetting step.

In particular for DNA library preparation preferably a second DNA-adapter-molecule is ligated to the other end of the fragmented, end repaired and 5' phosphorylated double stranded genomic DNA. This second DNA-adapter-molecule preferably contains reverse primer sequences and is preferably a universal adapter molecule, which is the same for every probe. The second DNA-adapter-molecule is either a DNA-adapter-molecule according to the invention or a common DNA-adapter-molecule as used in the state of the art also comprising a double-stranded polynucleotide molecule. The second DNA-adapter-molecule preferably comprises nucleotides with a modified backbone (as phosphor thioates).

Preferably the second DNA-adapter-molecule is a DNA-adapter-molecule according to the invention (with the 5' ends of first and reverse strand and the 3' end of the first strand modified as described above). As universal adapter molecule the second DNA-adapter-molecule does not need to contain a bar code, but preferably contains reverse primer sequences. Advantageously it can also be added after step 1.

If the second DNA-adapter-molecule (or universal adapter molecule) is a DNA-adapter-molecule according to the invention, this has the advantage that no inactivation of enzymes, in particular end repair enzymes is needed, before performing the ligation.

If the second DNA-adapter-molecule (or universal adapter molecule) is a common DNA-adapter-molecule known from the state of the art an inactivation of end repair enzymes has to be performed before ligation (step 4), which is preferably done by incubation at elevated temperature, which depends on the enzyme used, preferably a temperature over 60°C. In this case the second DNA-adapter-molecule is added after inactivation, preferably with the ligase in the ligation mix.

As steps 1 to 3 can be performed independently in another lab, the invention comprises also a method for the generation of a DNA library comprising the step of ligation of DNA-adapter-molecules according to the invention to fragmented, end repaired and 5' phosphorylated double stranded DNA, whereat preferably no inactivation of enzymes, in particular end repair enzymes, is performed before ligation.

The ligation is a blunt end ligation, which is preferably performed at conditions well known in the art (e.g. 2 h at RT or 16°C overnight). The preferred enzyme used for the ligation is T4 DNA ligase.

The ligation step as explained above is preferably followed by a nick repair (step 5) by adding a nick repair enzyme, preferably a DNA dependent DNA polymerase with 5'->3' exonuclease activity, and 5'->3' polymerase activity. The nick repair enzyme is preferably thermostable, like e.g. Taq polymerase. By preferably heating up the reaction mix, preferably to temperatures above 60°C, the ligase is inactivated while the end repair enzyme is activated. The enzymes used for step 4 and step 5 can be added simultaneously as a mix - "ligation and nick repair enzyme mix", containing the nick repair enzyme and ligase. The buffering agent used for step 4 and step 5 "as ligation buffer" can be advantageously the same as in the "end repair buffer", like TRIS-HCI and preferably with a pH between 7 and 8. The "ligation buffer" preferably also contains magnesium. For nick repair deoxynucleoside triphosphates (preferably dATP, dTTP, dGTP and dCTP) are also added either to the "ligation buffer" or separately.

The DNA used for generating the DNA library is preferably genomic DNA. One of the significant features of the genomic DNA library resides in that the genomic DNA library substantially maintains the copy numbers of a set of genes or sequences on the original genome (the genomic DNA) and the abundance ratio of the set of genes or sequences on the genomic DNA.

Another object of the invention is a kit for generating a DNA library, preferably for performing the method for generating a DNA library described above, comprising:
**i.** DNA-adapter-molecules according to the invention,
**ii.** preferably for the end repair: DNA polymerase with 3'->5'exonuclease and 5'->3' polymerase activity as well as deoxynucleoside triphosphates (preferably dATP, dTTP, dGTP and dCTP),
**iii.** preferably for the 5' phosphorylation: a polynucleotide kinase,
**iv.** preferably for the ligation: a ligase,
**v.** preferably a second DNA-adapter-molecule (preferably a universal adapter) as described above,
**vi.** preferably for the nick repair: a polymerase with 5'->3' exonuclease activity, and
**vii.** preferably buffer (as described above preferably comprising magnesium and ATP).

Thus the kit comprises at least DNA-adapter-molecules according to the invention and most preferred a ligase.

The different enzymes are preferably selected as described above.

Components ii and iii are preferably provided as "end repair enzyme mix" as defined above, preferably together with an "end repair buffer" as defined above or even mixed with this buffer.

Components iv and v are preferably provided as "ligation and nick repair enzyme mix" end repair enzyme mix" preferably together with an "ligation buffer" as defined above or even mixed with this buffer.

If not provided in the buffers, the kit might also contain deoxynucleoside triphosphates (preferably dATP, dTTP, dGTP and dCTP) as "dNTP mix" as separate component.

Also part of the invention is the use of the DNA-adapter-molecules described above or the kit described above for generating a DNA library, in particular for use in the field of Next Generation Sequencing and/or Library Multiplexing. The DNA-adapter-molecules according to the invention are particularly suitable for the use with automated solutions, since they allow for very simple and robust protocols.

The DNA-adapter modifications according to the invention enable and facilitate Library Multiplexing, especially in automated protocols, since the barcoded adapters can already be added manually to the DNA at the beginning of the library preparation (to the fragmented DNA, before the end repair and the phosphorylation of 5' ends is performed) and subsequently the library preparation can be carried out on a pipetting robot. A further advantageous characteristic is, that heat-inactivation of the end repair enzymes is not necessary: The end-repaired library fragments are not further modified by the enzymes, being present in the end-repair-mix and the DNA-adapter-molecules according to the invention are no substrate for those enzymes as well. An inactivation of those enzymes previous to the ligation before adding the DNA ligase is not necessary and the ligation can be carried out in the presence of active end repair enzymes. This allows for drastically shortened automated library preparation protocols without time consuming heating and cooling of the samples.

The invention is further described by the following figures and examples without being limited to them.

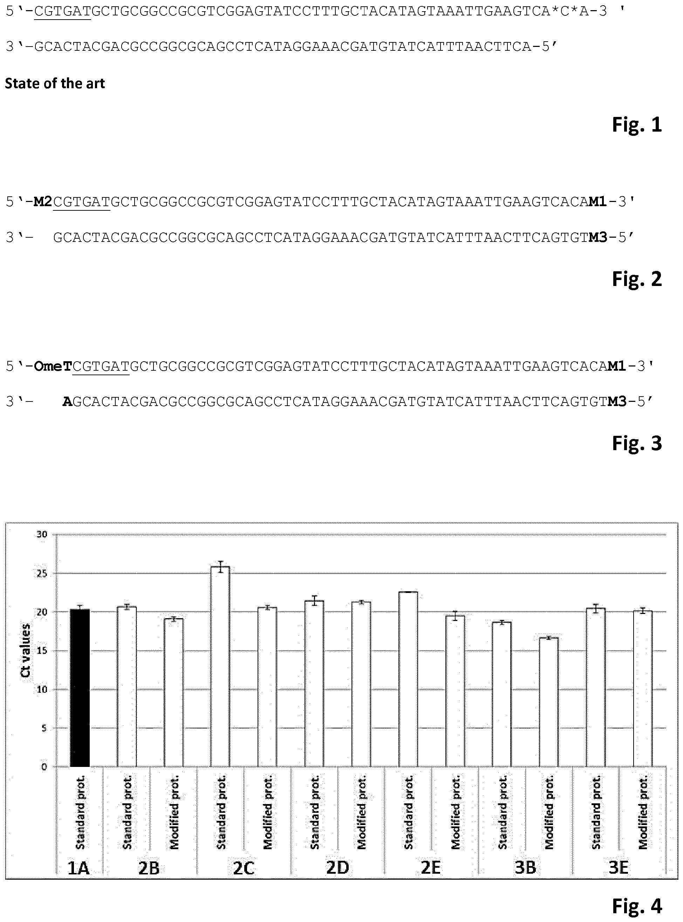

**Fig. 1** shows a barcode adapter known from the state of the art, which is optimized for blunt end ligation but cannot be added during or before end repair (and not without inactivation of end repair enzymes). On the left side the blunt ended ligation site is shown. To avoid ligation on the 3' site the first strand (SEQ ID No. 9) has a 3' overhang comprising the phosphor thioate modified nucleosides A*C* (to avoid degradation of the overhang). The complementary reverse strand (SEQ ID No. 10) is shorter. The 5' of the both strands are non-phosphorylated (have free 5'OH) to reduce formation of adaptor dimers. The barcode sequence is underlined.
**Fig. 2** shows an example of a barcode adapter according to the invention. Again on the left side the blunt ended ligation site is shown. To avoid ligation on the 3'site the first strand (SEQ ID No. 11) is modified by M1. The 5' of the first strand is modified by M2 to avoid phosphorylation and to reduce formation of adaptor dimers and oligomers. The 5' of the reverse strand (SEQ ID No. 12) is modified by M3 to reduce formation of adaptor dimers and oligomers. The barcode sequence is underlined. The 3' end of the reverse strand has a free 3'-OH
**Fig. 3** shows a modified barcode adapter according to the invention where the 5'-modification of the first strand (SEQ ID No. 13) is 5'-OMeT. For blunt end generation, the reverse strand (SEQ ID No. 14) has a complementary A at the 3' end which has a free 3'-OH group.
**Fig. 4** shows the comparison of Ct-values of DNA-libraries generated by using the standard protocol and new (or modified) library preparation protocol with unmodified and modiefed DNA-adapter-molecules, wherein 1A is the reference. The identifiers 1A to 3E correspond to the one out of table 1 (first column).

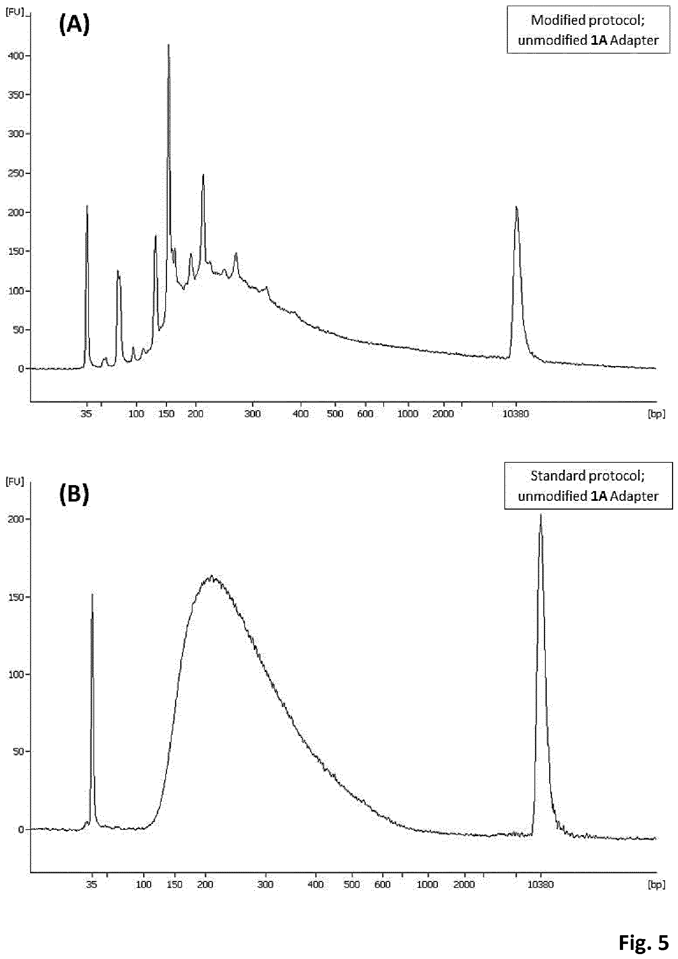

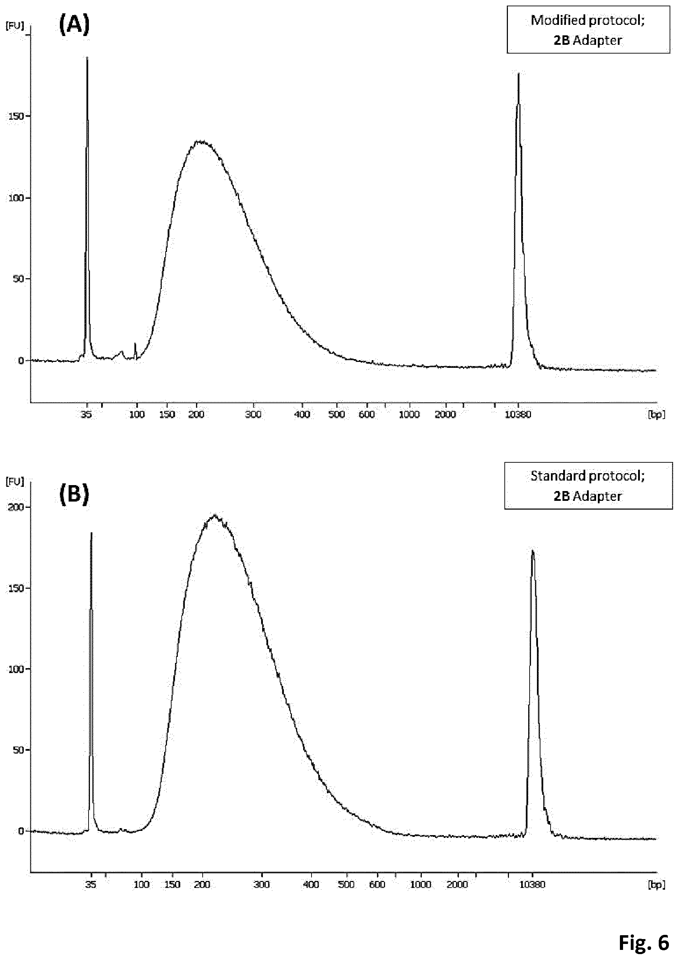

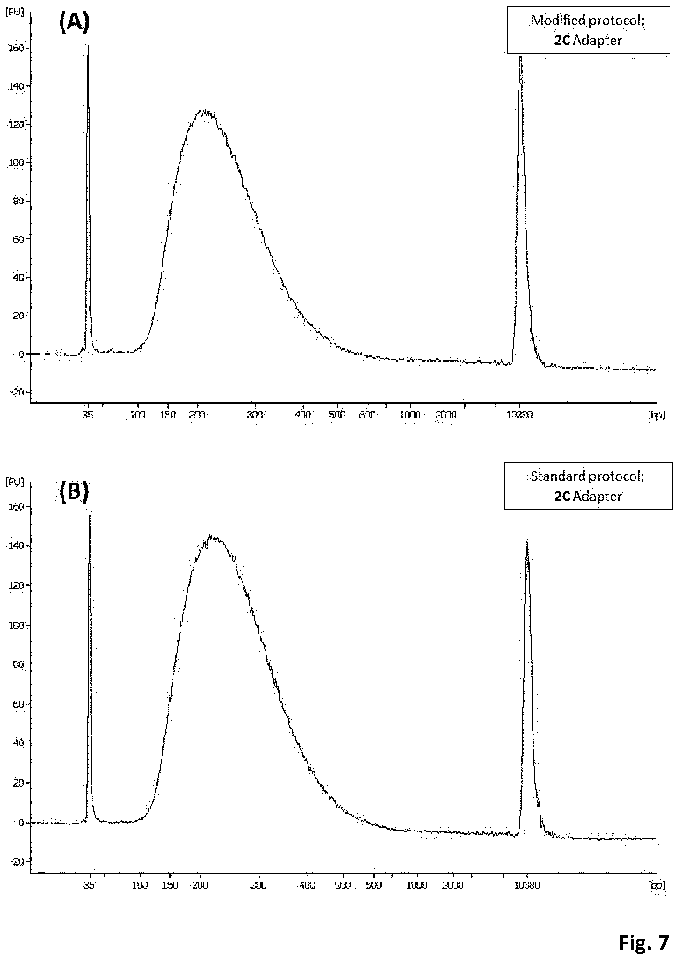

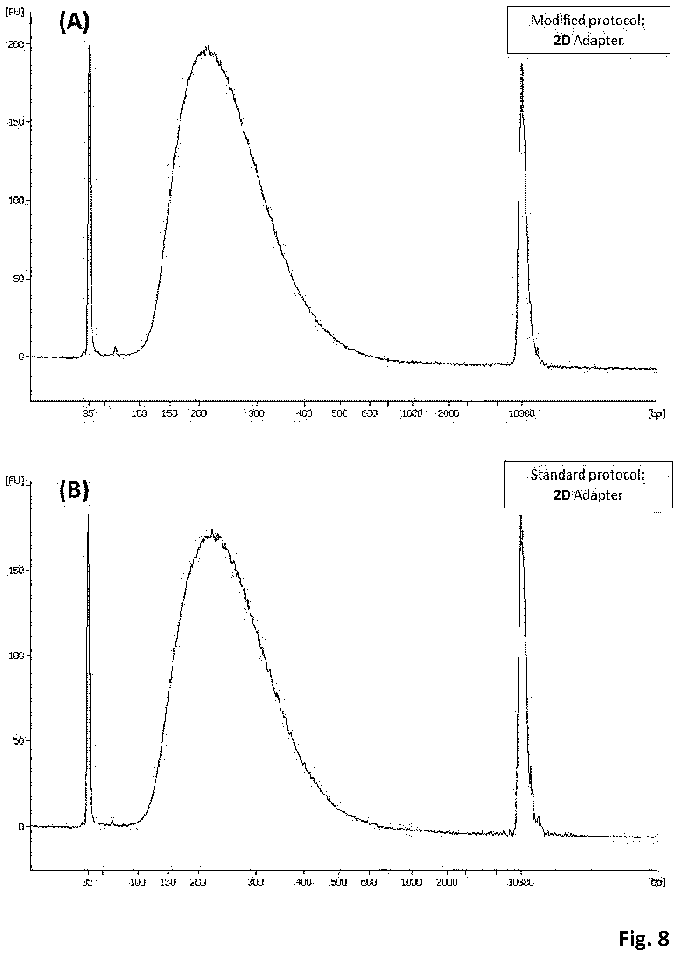

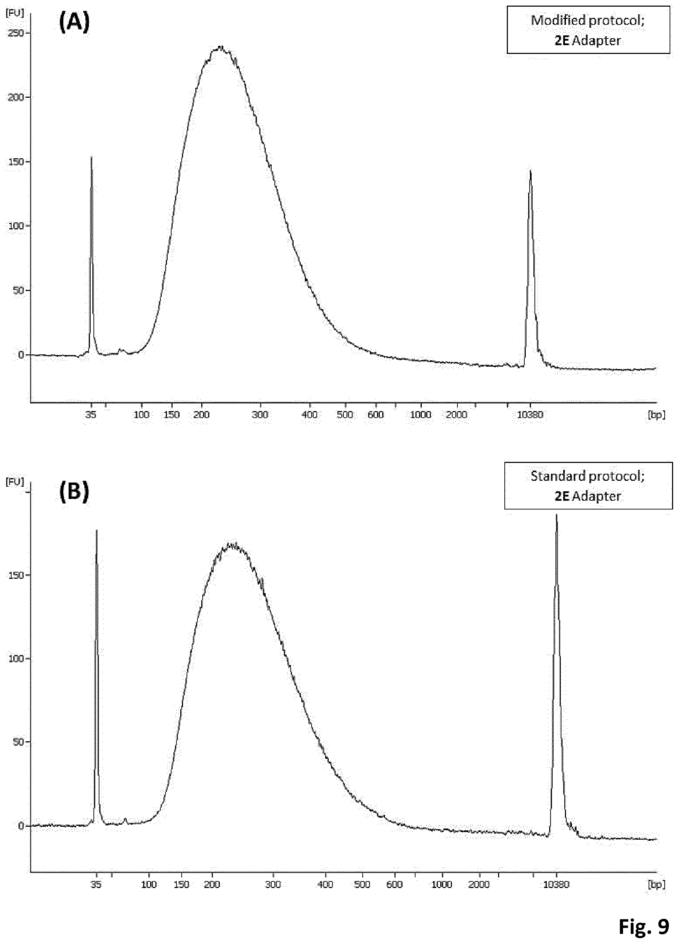

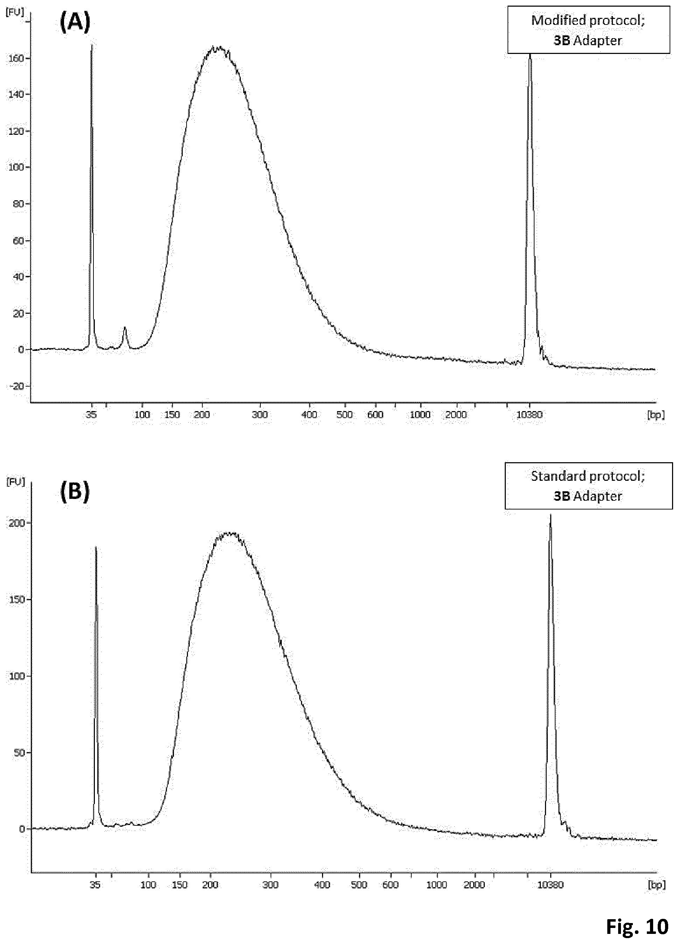

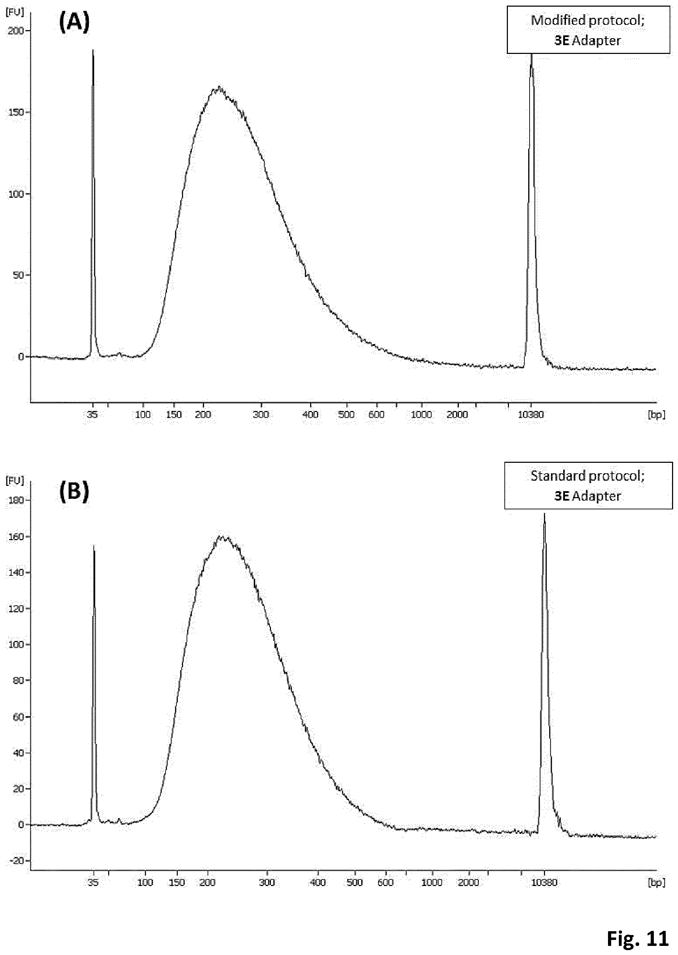

**Fig. 5 to 11** show the fragment size analysis of DNA libraries using modified barcode adapters according to the invention (Fig. 6 to 11) or unmodified barcode adapters (Fig. 5) either after standard library preparation **(B)** or after library preparation with the modified protocol according to the invention **(A)**. The DNA libraries are qualified using an Agilent High Sensitivity Chip in the size range between the lower and upper size marker (35-10380 bp). The identifiers 1A to 3E correspond to the one out of table 1. The first peak (40 bp) corresponds to unbound adapter molecules. The second peak in Fig 5B and Fig. 6 to 11 corresponds to the DNA library. The peak over 10.000 bp corresponds to remaining unsheared genomic DNA. In Fig. 5A the multi peak pattern corresponds to adapter oligomers, which are not present in the other figures.

### Example 1: Comparison of differently modified DNA-adapter molecules in a standard protocol and in a modified library preparation protocol

For testing different combinations of DNA-adapter modifications the following single strands are synthesized and applied in different combinations:

**Table 1:**

| **Identifier** | | **Reverse strand (5'→3')** | |
|---|---|---|---|
| **1** | B2_ Index1_ unmod | TGTGACTTCAATTTACTATGTAGCAAAGGATACTCCGACGCGGCCGCAGCATCACGA | SEQ ID No.1 |
| **2** | B2_ Index1_ C3 | **/5SpC3/**TGTGACTTCAATTTACTATGTAGCAAAGGATACTCCGACGCGGCCGCAGCATCACGA | SEQ ID No. 2 |
| **3** | B2_ Index1_ spacer | **/5dSp/**TGTGACTTCAATTTACTATGTAGCAAAGGATACTCCGACGCGGCCGCAGCATCACGA | SEQ ID No. 3 |

| | | **First Strand** | |
|---|---|---|---|
| **A** | B2_ Com_ Index1_ unmod | TCGTGATGCTGCGGCCGCGTCGGAGTATCCTTTGCTACATAGTAAATTGAAGTCACA | SEQ ID No.4 |
| **B** | B2_ Comp_ Index1_ C3_ Amino | **/5SpC3/**TCGTGATGCTGCGGCCGCGTCGGAGTATCCTTTGCTACATAGTAAATTGAAGTCACA **/3AmMO/** | SEQ ID No. 5 |
| **C** | B2_ Comp_ Index1_ C3_C3 | **/5SpC3/**TCGTGATGCTGCGGCCGCGTCGGAGTATCCTTTGCTACATAGTAAATTGAAGTCACA **/3SpC3/** | SEQ ID No. 6 |
| **D** | B2_ Comp_ Index1_ OMeT_ Amino | **5'- OMeT/**TCGTGATGCTGCGGCCGCGTCGGAGTATCCTTTGCTACATAGTAAATTGAAGTCACA**/3 AmMO/** | SEQ ID No. 7 |
| **E** | B2_ Comp_ Index1_ OMeT_ C3 | **5'- OMeT/**TCGTGATGCTGCGGCCGCGTCGGAGTATCCTTTGCTACATAGTAAATTGAAGTCACA**/3 SpC3/** | SEQ ID No. 8 |

The term "/5SpC3/" refers to a 5'-C3-Spacer as described above, as defined herein, the 5'-C3-Spacer is 1-hydroxypropyl-3-phosphatidyl terminator of the following formula:

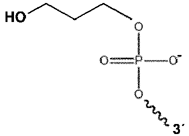

The term "/5dSp/" refers to 1,2-dideoxyribosyl-3-phosphate as a 5'-D-Spacer of the following formula:

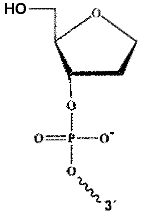

Herein the term "/3AmMO/" refers to 6-aminohexyl-1-phosphate as a 3'-Amino-Modifier of the following formula:

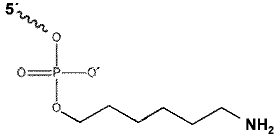

Herein "/3SpC3/" is 1-oxy-3-propanol as a 3'-C3-Spacer of the following formula:

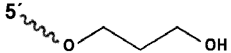

Herein "OMeT /" is the 5'-O-methyl deoxythymidine monophosphate as a 5'-end modifier of the following chemical structure:

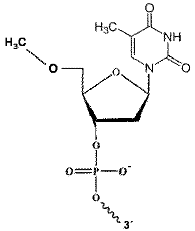

Different combinations of modified adapter strands are used in parallel to unmodified adapter-sequences (1A) in a DNA library preparation standard protocol (QIAGEN GeneRead™ Library Prep (L) Handbook 03/2013). For this, 1µg of genomic DNA (gDNA) from *E.coli* per preparation, which have been fragmented to a length of 150 base pairs before, are applied in an end repair reaction. Subsequently the enzymes are being heat inactivated and the differently modified adapter combinations together with the nonmodified universal adapter X (first strand X_fwd: 5'-GTAAAACGACGGCCAGT-3', SEQ ID No. 15; reverse strand: X_rev: 5'-ACTGGCCGTCGTTTTAC*T*T-3', SEQ ID No. 16; * =phosphor thioate) are applied in the ligation reaction.

In the present example the following adapter-combinations according to table 1 are used: 1A (unmodified adapter - state of the art), 2B, 2C, 2D, 2E, 3B, 3E.

Parallel to the standard protocol a modified library preparation protocol with the same combinations of adapter-molecules is carried out. In contrast to the standard protocol the barcode adapter B2_lndex_1 is pipetted together with the gDNA into the end-repair reaction. After finishing of the end-repair reaction only the universal adapter X is added to the ligation.

In the following table the two library preparation protocols ("new protocol" and "standard protocol") are illustrated:

**Table 2:**

| | Standard Protocol (volume in µl) state of the art | New Protocol (volume in µl) according to the invention |
|---|---|---|
| **1. End Repair** | | |
| Fragmented DNA | 18 | 18 |
| H₂O | 2.5 | 0.9 |
| Barcode-Adapter B2_Index_1 | 0.0 | 1.6 |
| End-Repair Buffer containing dNTPs, 10X | 2.5 | 2.5 |
| End-Repair enzyme mix | 2 | 2 |
| **Total Reaction Volume:** | 25 | 25 |
| | | |

| **2. Adapter Ligation** | | |
|---|---|---|
| End-repaired DNA in reaction mix | 25 | 25 |
| Ligation Buffer, 2X | 40 | 40 |
| Universal Adapter X | 1.6 | 1.6 |
| Barcode-Adapter B2_Index_1 | 1.6 | 0.0 |
| Ligation and Nick Repair mix | 4 | 4 |
| dNTP mix (10 mM) | 1 | 1 |
| H₂O | 6.8 | 8.4 |
| **Total Volume:** | 80 | 80 |

After the preparation of the End-Repair reaction the mixture is incubated for 20 minutes at 25°C followed by an enzyme inactivation step for 10 minutes at 70°C. Next, the ligation and nick repair reaction is prepared by adding Ligation and Nick Repair Mix, Ligation buffer and dNTPs together with the universal adapter to the end-repaired DNA. This mixture is incubated for 10 minutes at 25°C followed by incubation for 5 minutes at 72°C.

The resulting libraries are purified with the GeneRead™ size selection (QIAGEN, Hilden, DE, 100bp cutoff). After purification the resulting libraries are quantified via quantitative real time PCR (qPCR) with the aid of adapter-located primers and the fragment size distribution of the samples is analyzed using capillary gel electrophoresis.

The comparison of the Ct-values of the DNA libraries generated by using the standard protocol is showing no significant difference between the unmodified adapter-molecule 1A and the different adapter modifications (except adapter 2C). This fact is illustrated in Figure 3. It shows that the adapter modifications according to the invention do not influence the functionality of the adapter molecule.

The comparison of the Ct-values of the DNA libraries generated by using the standard protocol compared to those by using the new protocol is showing that the Ct-values for the modified adapter molecules after the new protocol are comparable or slightly better than after the standard protocol. The unmodified adapter molecule delivers significantly worse Ct-values when using the new protocol compared to the standard protocol, which shows that the yield under use of the modified adapter molecules according to the invention is independent of the applied library-preparation-protocol comparably high.

The fragment size distribution of the resulting DNA-libraries is compared with Agilent BioAnalyzer High Sensitivity DNA Chips. The fragment size distributions of the resulting DNA-libraries with unmodified (state of art) and modified DNA adapters according to the invention for the standard protocol are comparable. The fragment sizes are on average about 240 bp (150 bp of the library fragment after fragmentation + 90 bp of the adapter molecules). The same is valid for libraries which are resulting from using modified DNA adapter molecules according to the invention with the new protocol.

Only for libraries which are resulting from using unmodified adapter molecules with the new protocol clear peaks in the region of lower fragment sizes are visible in the electropherogram. This shows (Fig. 5A) that the unmodified adapter molecules are dimerizing and oligomerizing when being applied in the new protocol and thus are not applicable for the new protocol.

Adapter dimers and oligomers would falsify library quantification while quantitative PCR and one the other hand they would reduce the reading capacity when sequencing the library, because they are amplified and sequenced as eh DNA library, without containing any useful information.

## Claims

1. DNA-adapter-molecule, comprising a blunt-ended double-strand molecule, whereat
a. the 5' end of the first strand is modified in a way, that the first nucleotide does not contain a free hydroxyl group and no free phosphate at the 5' position,
b. the 3' end of the first strand is modified in a way that the last nucleotide does not contains a free hydroxyl group at the 3' position,
c. the 5' end of the reverse strand is modified in a way, that the first nucleotide does not contain free hydroxyl group and no free phosphate at the 5' position,
d. the 3' end of the reverse strand features a free hydroxyl group.

2. DNA-adapter-molecule according to claim 1, containing a barcode sequence as well as binding sites for amplification and sequencing primers.

3. DNA-adapter-molecule according to claim 1 or 2, **characterized in that** it has a length of 20 to 90, preferred of 40 to 70, most preferred of 40 to 60 base pairs.

4. DNA-adapter-molecule according to one of the claims 1 to 3, **characterized in that** the hydroxyl groups at the 5' ends of both strands and at the 3' end of the first strand are esterified or etherified.

5. DNA-adapter-molecule according to one of the claims 1 to 4, **characterized in that** the 5' end of the reverse strand is modified by attachment of 5'-C3-spacer or 5'-D-spacer.

6. DNA-adapter-molecule according to one of the claims 1 to 5, **characterized in that** the 5' end of the first strand is modified by attachment of 5' C3-spacer or a 5'- O-methyl deoxynucleotide

7. DNA-adapter-molecule according to one of the claims 1 to 6, **characterized in that** the 3' end of the first strand is modified by 3'-C3-spacer or 3'-amino-modifier.

8. Method for the production of DNA-adapter-molecules, comprising a blunt-ended double-strand molecule with the following steps:
a. modification of the 5' end of the first strand in a way, that no free hydroxyl group, and no free phosphate is available,
b. modification of the 3' end of the first strand in a way that no free hydroxyl group is available,
c. modification of the 5' end of the reverse strand in a way, that no free hydroxyl group, and no free phosphate is available.

9. Method for the production of DNA-adapter-molecules according to claim 8, whereat the modification of the 5' end of the first strand is achieved by attachment of 5'-O-methyl deoxythymidine monophosphate or a 5'-C3-spacer.

10. Method for the production of DNA-adapter-molecules according to claim 8 or 9, whereat the modification of the 3' end of the first strand is achieved by attachment of 3'-C3-spacer or 3'-amino-modifiers.

11. Method for the production of DNA-adapter-molecules according to one of the claims 8 to 10, whereat the modification of the 5' end of the reverse strand is achieved by attachment of 5'-C3-spacer or 5'-D-spacer.

12. Method for generation of a DNA library, comprising the steps
1. fragmentation of double stranded DNA, preferably by physical or chemical or enzymatic methods or a combination thereof,
2. end repair of the fragmented double stranded DNA, preferably with the aid of a DNA polymerase with 3'->5'exonuclease and 5'->3' polymerase activity, like T4-DNA polymerase,
3. phosphorylation of 5' ends of the fragmented double stranded DNA, preferably by a polynucleotide kinase, like T4 polynucleotide kinase,
4. ligation of blunt-ended DNA-adapter-molecules according to any one of claims 1-7 to the fragmented, end repaired and 5' phosphorylated double stranded DNA, whereat the DNA-adapter-molecules according to any one of claims 1-7 are added before step 2.

13. Method for generation of a DNA library, comprising the step of ligation of blunt-ended DNA-adapter-molecules according to one of the claims 1 to 7 to fragmented, end repaired and 5' phosphorylated double stranded DNA, whereat no inactivation of enzymes is performed before ligation.

14. Kit comprising - blunt-ended DNA-adapter-molecules according to one of the claims 1 to 7,
- preferably DNA polymerase with 3'->5'exonuclease and 5'->3' polymerase activity as well as desoxynucleoside triphosphates,
- preferably a polynucleotide kinase,
- preferably ligase,
- polymerase with 5'->3' exonuclease activity, and
- preferably buffer.

15. Use of the blunt-ended DNA-adapter-molecules according to one of the claims 1 to 7 or kit according to claim 14 for the generation of a DNA library, in particular in the field of Next Generation Sequencing and/or Library Multiplexing.

## Patentansprüche

1. DNA-Adaptermolekül, umfassend ein doppelsträngiges Molekül mit stumpfem Ende, wobei
a. das 5'-Ende des ersten Strangs auf eine Weise modifiziert ist, dass das erste Nukleotid keine freie Hydroxylgruppe und kein freies Phosphat an der 5'-Position enthält,
b. das 3'-Ende des ersten Strangs auf eine Weise modifiziert ist, dass das letzte Nukleotid keine freie Hydroxylgruppe an der 3'-Position enthält,
c. das 5'-Ende des reversen Strangs auf eine Weise modifiziert ist, dass das erste Nukleotid keine freie Hydroxylgruppe und kein freies Phosphat an der 5'-Position enthält,
d. das 3'-Ende des reversen Strangs eine freie Hydroxylgruppe aufweist.

2. DNA-Adaptermolekül nach Anspruch 1, das eine Barcode-Sequenz sowie Bindungsstellen für Amplifikations- und Sequenzierungsprimer enthält.

3. DNA-Adaptermolekül nach Anspruch 1 oder 2, **dadurch gekennzeichnet, dass** es eine Länge von 20 bis 90, bevorzugt 40 bis 70, am stärksten bevorzugt 40 bis 60 Basenpaaren aufweist.

4. DNA-Adaptermolekül nach einem der Ansprüche 1 bis 3, **dadurch gekennzeichnet, dass** die Hydroxylgruppen an den 5'-Enden beider Stränge und an den 3'-Enden des ersten Strangs verestert oder verethert sind.

5. DNA-Adaptermolekül nach einem der Ansprüche 1 bis 4, **dadurch gekennzeichnet, dass** das 5'-Ende des reversen Strangs durch Anheften von 5'-C3-Spacer oder 5'-D-Spacer modifiziert ist.

6. DNA-Adaptermolekül nach einem der Ansprüche 1 bis 5, **dadurch gekennzeichnet, dass** das 5'-Ende des ersten Strangs durch Anheften eines 5'-C3-Spacers oder eines 5'-O-Methyldesoxynukleotids modifiziert ist.

7. DNA-Adaptermolekül nach einem der Ansprüche 1 bis 6, **dadurch gekennzeichnet, dass** das 3'-Ende des ersten Strangs durch einen 3'-C3-Spacer oder einen 3'-Amino-Modifikator modifiziert ist.

8. Verfahren zum Herstellen von DNA-Adaptermolekülen, die ein doppelsträngiges Molekül mit stumpfem Ende umfassen, mit den folgenden Schritten:
a. Modifizieren des 5'-Endes des ersten Strangs auf eine Weise, dass keine freie Hydroxylgruppe und kein freies Phosphat verfügbar sind,
b. Modifizieren des 3'-Endes des ersten Strangs auf eine Weise, dass keine freie Hydroxylgruppe verfügbar ist,
c. Modifizieren des 5'-Endes des reversen Strangs auf eine Weise, dass keine freie Hydroxylgruppe und kein freies Phosphat verfügbar sind.

9. Verfahren zum Herstellen von DNA-Adaptermolekülen nach Anspruch 8, wobei die Modifikation des 5'-Endes des ersten Strangs durch Anheften von 5'-O-Methyldesoxythymidinmonophosphat oder einem 5'-C3-Spacer erreicht wird.

10. Verfahren zum Herstellen von DNA-Adaptermolekülen nach Anspruch 8 oder 9, wobei die Modifikation des 3'-Endes des ersten Strangs durch Anheften von 3'-C3-Spacer oder 3'-Amino-Modifikatoren erreicht wird.

11. Verfahren zum Herstellen von DNA-Adaptermolekülen nach einem der Ansprüche 8 bis 10, wobei das Modifizieren des 5'-Endes des reversen Strangs durch Anheften von 5'-C3-Spacer oder 5'-D-Spacer erreicht wird.

12. Verfahren zum Erzeugen einer DNA-Bibliothek, die folgenden Schritte umfassend:
1. Fragmentieren von doppelsträngiger DNA, vorzugsweise durch physikalische oder chemische oder enzymatische Verfahren oder eine Kombination daraus,
2. Endreparieren der fragmentierten doppelsträngigen DNA, vorzugsweise mit der Hilfe einer DNA-Polymerase mit 3'->5'-Exonuklease- und 5'->3'-Polymeraseaktivität, wie T4-DNA-Polymerase,
3. Phosphorylieren von 5'-Enden der fragmentierten doppelsträngigen DNA, vorzugsweise durch eine Polynukleotidkinase, wie T4-Polynukleotidkinase,
4. Ligieren von DNA-Adaptermolekülen mit stumpfem Ende nach einem der Ansprüche 1-7 an die fragmentierte, endreparierte und 5'-phosphorylierte doppelsträngige DNA, wobei die DNA-Adaptermoleküle nach einem der Ansprüche 1-7 vor Schritt 2 hinzugefügt werden.

13. Verfahren zum Erzeugen einer DNA-Bibliothek, umfassend den Schritt des Ligierens von DNA-Adaptermolekülen mit stumpfem Ende nach einem der Ansprüche 1 bis 7 an fragmentierte endreparierte und 5'-phosphorylierte doppelsträngige DNA, wobei keine Inaktivierung von Enzymen vor dem Ligieren durchgeführt wird.

14. Kit, Folgendes umfassend:
- DNA-Adaptermoleküle mit stumpfem Ende nach einem der Ansprüche 1 bis 7,
- vorzugsweise DNA-Polymerase mit 3'->5'-Exonuklease- und 5'->3'-Polymeraseaktivität sowie Desoxynukleosidtriphosphaten,
- vorzugsweise eine Polynukleotidkinase,
- vorzugsweise Ligase,
- Polymerase 5'->3'-Exonuklease-Aktivität, und
- vorzugsweise Puffer.

15. Verwendung der DNA-Adaptermoleküle mit stumpfem Ende nach einem der Ansprüche 1 bis 7 oder Kit nach Anspruch 14 zum Erzeugen einer DNA-Bibliothek, insbesondere im Gebiet der Sequenzierung der nächsten Generation und/oder des Bibliothek-Multiplexings.

## Revendications

1. Molécule adaptatrice d'ADN, comprenant une molécule double brin à extrémité franche, dans laquelle
a. l'extrémité 5' du premier brin est modifiée de manière à ce que le premier nucléotide ne contienne ni de groupe hydroxyle libre ni de phosphate libre à la position 5',
b. l'extrémité 3' du premier brin est modifiée de manière à ce que le dernier nucléotide ne contienne pas de groupe hydroxyle libre à la position 3',
c. l'extrémité 5' du brin antisens est modifiée de manière à ce que le premier nucléotide ne contienne ni de groupe hydroxyle libre ni de phosphate libre à la position 5',
d. l'extrémité 3' du brin antisens présente un groupe hydroxyle libre.

2. Molécule adaptatrice d'ADN selon la revendication 1, contenant une séquence de code-barres ainsi que des sites de liaison pour des amorces d'amplification et de séquençage.

3. Molécule adaptatrice d'ADN selon la revendication 1 ou 2, **caractérisée en ce qu'**elle a une longueur de 20 à 90, de préférence de 40 à 70 et de manière encore plus préférable de 40 à 60 paires de bases.

4. Molécule adaptatrice d'ADN selon l'une des revendications 1 à 3, **caractérisée en ce que** les groupes hydroxyle aux extrémités 5' des deux brins et à l'extrémité 3' du premier brin sont estérifiés ou éthérifiés.

5. Molécule adaptatrice d'ADN selon l'une des revendications 1 à 4, **caractérisée en ce que** l'extrémité 5' du brin antisens est modifiée par fixation du 5'-C3-espaceur ou du 5'-D-espaceur.

6. Molécule adaptatrice d'ADN selon l'une des revendications 1 à 5, **caractérisée en ce que** l'extrémité 5' du premier brin est modifiée par fixation du 5'-C3-espaceur ou d'un 5'-O-méthyl-désoxynucléotide.

7. Molécule adaptatrice d'ADN selon l'une des revendications 1 à 6, **caractérisée en ce que** l'extrémité 3' du premier brin est modifiée par le 3'-C3-espaceur ou le 3'-amino-modificateur.

8. Procédé de production de molécules adaptatrices d'ADN, comprenant une molécule double brin à extrémité franche avec les étapes suivantes :
a. la modification de l'extrémité 5' du premier brin de manière à ce qu'aucun groupe hydroxyle libre et phosphate libre ne soit disponible,
b. la modification de l'extrémité 3' du premier brin de manière à ce qu'aucun groupe hydroxyle libre ne soit disponible,
c. la modification de l'extrémité 5' du brin antisens de manière à ce qu'aucun groupe hydroxyle libre et phosphate libre ne soit disponible.

9. Procédé de production de molécules adaptatrices d'ADN selon la revendication 8, dans lequel la modification de l'extrémité 5' du premier brin est réalisée par fixation du 5'-O-méthyl-désoxythymidine monophosphate ou d'un 5'-C3-espaceur.

10. Procédé de production de molécules adaptatrices d'ADN selon la revendication 8 ou 9, dans lequel la modification de l'extrémité 3' du premier brin est réalisée par fixation du 3'-C3-espaceur ou des 3'-amino-modificateurs.

11. Procédé de production de molécules adaptatrices d'ADN selon l'une des revendications 8 à 10, dans lequel la modification de l'extrémité 5' du brin antisens est réalisée par fixation du 5'-C3-espaceur ou du 5'-D-espaceur.

12. Procédé de génération d'une banque d'ADN, comprenant les étapes suivantes :
1. la fragmentation d'ADN double brin, de préférence par des procédés physiques ou chimiques ou enzymatiques ou par une combinaison de ceux-ci,
2. la réparation des extrémités de l'ADN double brin fragmenté, de préférence à l'aide d'une ADN polymérase avec une activité exonucléase 3'->5' et polymérase 5'->3', comme l'ADN polymérase de T4,
3. la phosphorylation d'extrémités 5' de l'ADN double brin fragmenté, de préférence par une polynucléotide kinase, comme la polynucléotide kinase T4,
4. la ligature de molécules adaptatrices d'ADN à extrémité franche selon l'une quelconque des revendications 1 à 7 avec l'ADN double brin fragmenté, à extrémités réparées et à extrémité 5' phosphorylée, dans laquelle les molécules adaptatrices d'ADN selon l'une quelconque des revendications 1 à 7 sont ajoutées avant l'étape 2.

13. Procédé de génération d'une banque d'ADN, comprenant l'étape de ligature de molécules adaptatrices d'ADN à extrémité franche selon l'une des revendications 1 à 7 avec l'ADN double brin fragmenté, à extrémités réparées et à extrémité 5' phosphorylée, dans lequel aucune inactivation enzymatique n'est réalisée avant la ligature.

14. Kit comprenant
- des molécules adaptatrices d'ADN à extrémité franche selon l'une des revendications 1 à 7,
- de préférence de l'ADN polymérase avec une activité exonucléase 3'->5' et polymérase 5'->3' ainsi que des désoxynucléosides triphosphates,
- de préférence une polynucléotide kinase,
- de préférence de la ligase,
- de la polymérase avec une activité exonucléase 5'->3', et
- de préférence un tampon.

15. Utilisation des molécules adaptatrices d'ADN à extrémité franche selon l'une des revendications 1 à 7 ou kit selon la revendication 14 pour la génération d'une banque d'ADN, en particulier dans le domaine du séquençage de nouvelle génération et/ou du multiplexage de banques.


### SEQUENCE LISTING

<110> QIAGEN GmbH
<120> DNA-adapter-molecules for the preparation of DNA-libraries and method for producing them and use
<130> 00139P0010EPWO
<150> EP 13 186 776.4
   <151> 2013-09-30
<160> 16
<170> PatentIn version 3.5
<210> 1
   <211> 57
   <212> DNA
   <213> artificial
<220>
   <223> Universal DNA adaptor reverse strand
<400> 1
   tgtgacttca atttactatg tagcaaagga tactccgacg cggccgcagc atcacga 57
<210> 2
   <211> 58
   <212> DNA
   <213> artificial
<220>
   <223> Universal DNA adaptor reverse strand
<220>
   <221> modified_base
   <222> (1)..(1)
   <223> n is 1-hydroxypropyl-3-phosphatidyl
<400> 2
   ntgtgacttc aatttactat gtagcaaagg atactccgac gcggccgcag catcacga 58
<210> 3
   <211> 58
   <212> DNA
   <213> artificial
<220>
   <223> Universal DNA adaptor reverse strand
<220>
   <221> modified_base
   <222> (1)..(1)
   <223> n is 1,2-dideoxyribosyl-3-phosphate
<400> 3
   ntgtgacttc aatttactat gtagcaaagg atactccgac gcggccgcag catcacga 58
<210> 4
   <211> 57
   <212> DNA
   <213> artificial
<220>
   <223> Universal DNA adaptor forward strand
<400> 4
   tcgtgatgct gcggccgcgt cggagtatcc tttgctacat agtaaattga agtcaca 57
<210> 5
   <211> 59
   <212> DNA
   <213> artificial
<220>
   <223> Universal DNA adaptor forward strand
<220>
   <221> modified_base
   <222> (1)..(1)
   <223> n is 1-hydroxypropyl-3-phosphatidyl
<220>
   <221> modified_base
   <222> (59)..(59)
   <223> n is 6-aminohexyl-1-phosphate
<400> 5
   ntcgtgatgc tgcggccgcg tcggagtatc ctttgctaca tagtaaattg aagtcacan 59
<210> 6
   <211> 59
   <212> DNA
   <213> artificial
<220>
   <223> Universal DNA adaptor forward strand
<220>
   <221> modified_base
   <222> (1)..(1)
   <223> n is 1-hydroxypropyl-3-phosphatidyl
<220>
   <221> modified_base
   <222> (59)..(59)
   <223> n is 1-oxy-3-propanol
<400> 6
   ntcgtgatgc tgcggccgcg tcggagtatc ctttgctaca tagtaaattg aagtcacan 59
<210> 7
   <211> 59
   <212> DNA
   <213> artificial
<220>
   <223> Universal DNA adaptor forward strand
<220>
   <221> modified_base
   <222> (1)..(1)
   <223> n is 5'-O-methyl deoxythymidine monophosphate
<220>
   <221> modified_base
   <222> (59)..(59)
   <223> n is 6-aminohexyl-1-phosphate
<400> 7
   ntcgtgatgc tgcggccgcg tcggagtatc ctttgctaca tagtaaattg aagtcacan 59
<210> 8
   <211> 59
   <212> DNA
   <213> artificial
<220>
   <223> Universal DNA adaptor forward strand
<220>
   <221> modified_base
   <222> (1)..(1)
   <223> n is 5'-O-methyl deoxythymidine monophosphate
<220>
   <221> modified_base
   <222> (59)..(59)
   <223> n is 1-oxy-3-propanol
<400> 8
   ntcgtgatgc tgcggccgcg tcggagtatc ctttgctaca tagtaaattg aagtcacan 59
<210> 9
   <211> 56
   <212> DNA
   <213> artificial
<220>
   <223> Universal DNA adaptor forward strand
<220>
   <221> modified_base
   <222> (54)..(54)
   <223> n is phosphor thioate modified nucleoside A
<220>
   <221> modified_base
   <222> (55)..(55)
   <223> n is phosphor thioate modified nucleoside C
<400> 9
   cgtgatgctg cggccgcgtc ggagtatcct ttgctacata gtaaattgaa gtcnna 56
<210> 10
   <211> 52
   <212> DNA
   <213> artificial
<220>
   <223> Universal DNA adaptor reverse strand
<400> 10
   acttcaattt actatgtagc aaaggatact ccgacgcggc cgcagcatca cg 52
<210> 11
   <211> 58
   <212> DNA
   <213> artificial
<220>
   <223> Universal DNA adaptor forward strand
<220>
   <221> modified_base
   <222> (1)..(1)
   <223> n is etherified deoxynucleotide or 4'-Amino-deoxynucleotide or 5'-Amino-deoxynucleotide or 5'-C3-Spacer or 5'-D-Spacer
<220>
   <221> modified_base
   <222> (58)..(58)
   <223> n is 3'-Amino-Modifier or 3'-C3-Spacer
<400> 11
   ncgtgatgct gcggccgcgt cggagtatcc tttgctacat agtaaattga agtcacan 58
<210> 12
   <211> 57
   <212> DNA
   <213> artificial
<220>
   <223> Universal DNA adaptor reverse strand
<220>
   <221> modified_base
   <222> (1)..(1)
   <223> n is etherified deoxynucleotide or 4'-Amino-deoxynucleotide or 5'-Amino-deoxynucleotide or 5'-C3-Spacer or 5'-D-Spacer
<400> 12
   ntgtgacttc aatttactat gtagcaaagg atactccgac gcggccgcag catcacg 57
<210> 13
   <211> 58
   <212> DNA
   <213> artificial
<220>
   <223> Universal DNA adaptor forward strand
<220>
   <221> modified_base
   <222> (1)..(1)
   <223> n is 5'-O-methyl deoxythymidine monophosphate
<220>
   <221> modified_base
   <222> (58)..(58)
   <223> n is etherified deoxynucleotide or 4'-Amino-deoxynucleotide or 5'-Amino-deoxynucleotide or 5'-C3-Spacer or 5'-D-Spacer
<400> 13
   ncgtgatgct gcggccgcgt cggagtatcc tttgctacat agtaaattga agtcacan 58
<210> 14
   <211> 58
   <212> DNA
   <213> artificial
<220>
   <223> Universal DNA adaptor reverse strand
<220>
   <221> modified_base
   <222> (1)..(1)
   <223> n is etherified deoxynucleotide or 4'-Amino-deoxynucleotide or 5'-Amino-deoxynucleotide or 5'-C3-Spacer or 5'-D-Spacer
<400> 14
   ntgtgacttc aatttactat gtagcaaagg atactccgac gcggccgcag catcacga 58
<210> 15
   <211> 17
   <212> DNA
   <213> artificial
<220>
   <223> Universal DNA adaptor forward strand
<400> 15
   gtaaaacgac ggccagt 17
<210> 16
   <211> 19
   <212> DNA
   <213> artificial
<220>
   <223> Universal DNA adaptor reverse strand
<220>
   <221> n
   <222> (17)..(17)
   <223> n is 2'-Deoxycytosine-5'-O-monophosphorthioate
<220>
   <221> n
   <222> (18)..(18)
   <223> n is 2'-Deoxythymidine-5'-O-monophosphorthioate
<400> 16
   actggccgtc gttttannt 19